# EUROPEAN PATENT APPLICATION

(11) **EP 4 166 192 A1**
(43) Date of publication of application: **19.04.2023**
(21) Application number: 21202558.9
(22) Date of filing: 14.10.2021
(51) Int. Cl.: A61N 5/06, A61B 18/20, A61B 5/00, A61N 5/067, G06T 7/70, G06T 7/00, G06T 7/10

(54) **AN APPARATUS FOR USER GUIDANCE DURING SKIN TREATMENT AND METHOD THEREOF**

(71) Applicant: Koninklijke Philips N.V., 5656 AG Eindhoven (NL)
(72) Inventor: ABERNETHY, Simon George, Eindhoven (NL)
(74) Representative: Philips Intellectual Property & Standards

(57) **Abstract**

An apparatus for guiding a user during a skin treatment process is provided, and a method thereof. The apparatus comprises a treatment applier, a mapping unit which is configured to obtain a map of a skin area to be treated and a reference position on a boundary of the map, and a processor which is configured to divide an area of the obtained map into a plurality of compartments based on at least an area of the treatment applier, register contact of the treatment applier at the reference position and guide the user, via a user interface unit, through the compartments in the map based on displacement of the treatment applier relative to the reference position.

## Description

### FIELD OF THE INVENTION

The present invention relates to a skin treatment process, and in particular, to a control method and apparatus for guiding a user through the skin treatment process.

### BACKGROUND OF THE INVENTION

There are currently numerous skin treatment devices available to a user for domestic use. It is important that these devices are used safely at home, where the user often receives no guidance from a medical professional. Skin treatment devices also include devices which involve phototherapy or photo-epilation. When the radiation is not visible to the user, which for example is the case with infrared radiation, the medical safety of the device becomes even more relevant.

One of the solutions to ensure medical safety is by preventing overlap of treated skin areas or surfaces. Such overlap could cause injury to skin, as well as result in unevenly treated skin areas. It is therefore an object of the invention to assist a user of the device to manipulate the device in a safe manner by avoiding excessive treatment of skin.

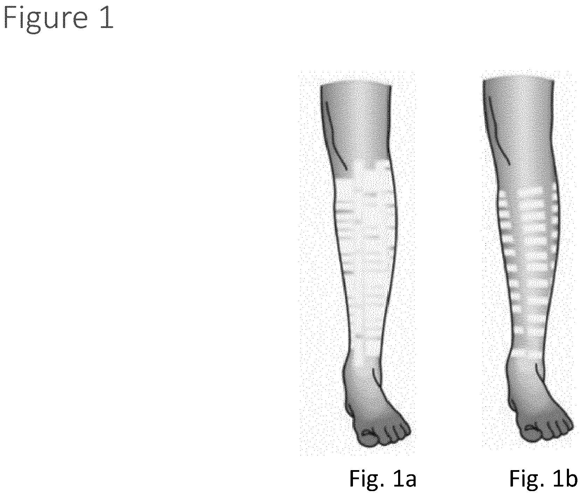

Fig. 1a shows a prior art situation where certain parts of the treated areas are treated multiple times, thus resulting in an over treatment of skin.

It is yet another object of the invention to provide a uniform coverage of treatment, not leaving untreated areas after completion of a treatment process. This not only improves the appearance of skin after treatment but also provides an improved user experience of the device.

Fig. 1b shows another prior art situation where certain parts of skin are left untreated, thus resulting in an under treatment of skin.

Many details below relate to light-based skin treatment devices, such as intense pulsed light (IPL) devices, however, the invention is not limited to a certain type of skin treatment.

### SUMMARY OF THE INVENTION

According to an aspect of the invention, an apparatus for guiding a user during a skin treatment process is provided. The apparatus comprises a treatment applier, a mapping unit which is configured to obtain a map of a skin area to be treated and a reference position on a boundary of the map, and a processor which is configured to divide an area of the obtained map into a plurality of compartments based on at least an area of the treatment applier, register contact of the treatment applier at the reference position and guide the user, via a user interface unit, through the compartments in the map based on displacement of the treatment applier relative to the reference position.

According to another aspect of the invention, a computer-implemented method is provided, for guiding a user during a skin treatment process. The process may be cosmetic or medical, and as will be clear from below, the advantages/effects of the invention are independent of the type of treatment. An inextricably linked therapeutic effect is not foreseen according to the invention. The method comprises obtaining a map of a skin surface to be treated and a reference position on a boundary of the map, dividing an area of the obtained map into a plurality of compartments based on at least an area of a treatment applier, registering contact of the treatment applier at the reference position and guiding the user through the compartments in the map based on displacement of a treatment applier relative to the reference position. The method is further characterized by steps carried out by an apparatus of the present invention as described below.

According to yet another aspect of the invention, a computer program product, which comprises a computer readable medium having computer readable code embodied therein, the computer readable code being configured such that, on execution by a suitable computer or processor, the processor is caused to perform the method described below.

As a result, the invention provides a reliable user guidance solution which avoids excessive or under treatment of skin.

These and other aspects, and further advantages, will be apparent from and elucidated with reference to the embodiment(s) described herein.

### BRIEF DESCRIPTION OF THE DRAWINGS

Figs. 1a and 1b show a skin treatment process using a prior art apparatus.
Fig. 2 shows a block diagram of the apparatus according to an embodiment of the invention.
Fig. 3 shows a map of the proposed treatment area according to an embodiment of the invention.
Fig. 4 shows an apparatus 400 according to an embodiment of the invention.
Fig. 5 shows a method for controlling apparatus 200 or 400 according to an embodiment of the invention.

### DETAILED DESCRIPTION OF THE EMBODIMENTS

The matters exemplified in this description are provided to assist in a comprehensive understanding of various exemplary embodiments of the present invention disclosed with reference to the accompanying figures.

Those of ordinary skill in the art will recognize that various changes and modifications of the exemplary figures/embodiments described herein can be made without departing from the scope of the claimed invention. In particular, combinations of specific features of various aspects or embodiments of the invention may be made. An aspect or embodiment of the invention may be further advantageously enhanced by adding a feature that was described in relation to another aspect or embodiment of the invention.

The terms and words used in the following description and claims are not limited to their dictionary meanings, but are merely used to enable a clear and consistent understanding of the present disclosure.

Further, the functionality associated with any particular means may be centralized or distributed, whether locally or remotely. A computer program may be stored/distributed on a suitable medium, such as an optical storage medium or a solid-state medium supplied together with or as part of other hardware, but may also be distributed in other forms, such as via the Internet or other wired or wireless telecommunication systems.

It may be advantageous to set forth that the terms "include" and "comprise," as well as derivatives thereof, mean inclusion without limitation. In addition, reference to an element by the indefinite article "a" or "an" does not exclude the possibility that more than one element is present, unless the context clearly requires that there be one and only one of the elements. The indefinite article "a" or "an" thus usually means "at least one". For example, a treatment compartment includes reference to one or more of the treatment compartments.

The expression "at least one of A, B and C" means "A, B, and/or C", and that it suffices if for example only B is present. Any reference signs in the claims should not be construed as limiting the scope.

Terms such as, for example, "processing," "computing," "calculating," "determining," "establishing", "analyzing", "checking", or the like, used herein, may refer to, in a non-limiting manner, operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium (e.g., a memory) that may store instructions to perform operations and/or processes. The terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, unless otherwise specified, some of the described method embodiments or elements thereof can occur or be performed simultaneously, at the same point in time, or concurrently.

The term "user" is used to refer to the user of the apparatus or method, and to a subject on which the apparatus or method is used.

The term "skin area" is used to refer to an area of a surface of the most external skin layer.

The term "compartment" is used to refer to a portion of a certain total area which is divided from the total area. A total area of N units may be divided into n compartments.

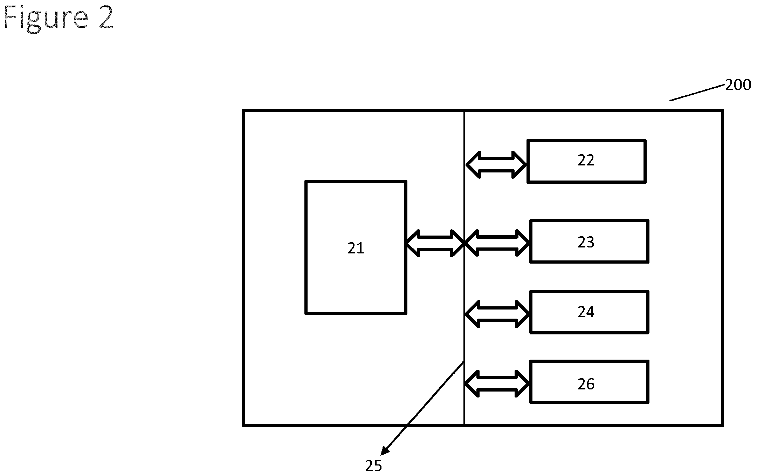

Fig. 2 shows a block diagram of an apparatus 200 according to an embodiment of the invention.

The apparatus 200 is typically hand-held and comprises a treatment applier 20 which can be used to apply treatment to the skin. In an aspect, the treatment applier 20 is a portion of the skin treatment apparatus 200 which is brought in contact or proximity of skin. In an aspect, the treatment applier 20 is a treatment window of the apparatus 200. If the skin treatment apparatus 200 is light-based, the treatment applier 20 may be an arrangement of an attachment including an optical treatment window which allows treatment light to be transmitted to the skin. In an aspect, the treatment applier 20 may be removably attached to the apparatus.

The apparatus 200 further comprises a mapping unit 21. In an embodiment, the mapping unit 21 maps a proposed treatment area, i.e., the skin area to be treated. The mapping unit 21 comprises a displacement sensor which may be an optical displacement sensor 201 or a mechanical displacement sensor 202.

The optical displacement sensor 201 includes a camera (e.g., CCD, CMOS) which can be used to obtain at least one image of a proposed treatment area. In an aspect, a pixel count registered by the camera may be converted to position information of the apparatus on the skin to generate the map.

The mechanical displacement sensor 202 may be a mechanical mouse sensor with rollers. The rotation of the rollers on the skin may be converted to position information of the apparatus on the skin to generate the map.

In an aspect, the mapping unit 21 can map the skin area to be treated by directly photographing/imaging the proposed treatment area using an imaging unit, e.g., using a camera. The camera may be positioned at a suitable location in the apparatus 200 to be able to capture an image of the proposed treatment area with the desired field of view. The mapping unit 21 may then determine an arbitrary reference position on the boundary of the mapped treatment area. In an aspect, the camera may be an external camera, e.g., that of a smartphone which can be attached to the apparatus at a convenient position. The details of this implementation are included in (Euro-)PCT application PCT/EP2021/059613.

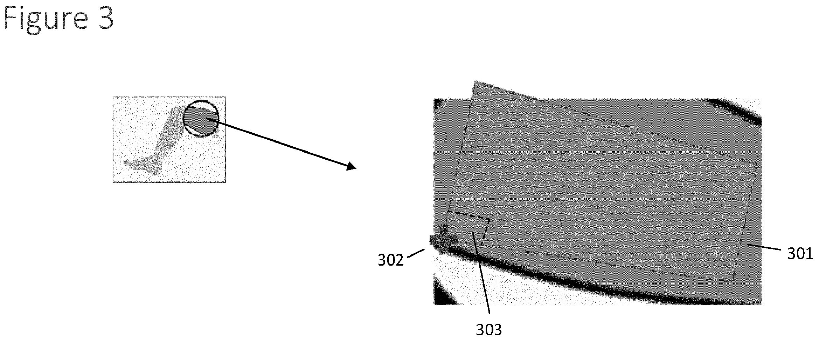

In another aspect, the user may manually map the treatment area by tracing a boundary of the proposed area while holding the apparatus. In doing so, the treatment applier is moved on the skin with the treatment source disabled. The mapping unit 21 senses a displacement of the treatment applier 20 on the skin from a start position (which may be regarded as the reference position) and converts its displacement from the start position to position information of the apparatus relative to the skin. The mapping unit 21 obtains the map of the proposed treatment area by accumulating the position information of the apparatus on the skin. Such a map of the proposed treatment area is shown in Fig. 3, where boundary 301 is traced by the user starting from reference position 302. A treatment compartment 303 is further shown. The memory 22 stores the map e.g., its characteristics (size, shape etc.) and the reference position 302, from where the mapping unit 21 retrieves or obtains it. Additional functions of memory 22 are detailed below.

The reference position 302 serves as a reference to the user regarding the initiation of treatment. Further, it is used by the mapping unit 21 and/or processor 23 to calculate displacement of the apparatus and its relative position on the skin as mentioned above, in order to guide the user through the skin treatment process.

In an aspect, it is possible to indicate to the user the reference position 302 via a user interface unit 24. A visual indicator such as an LED may be used for such indication. In another aspect, the boundary 301 may be visually indicated to the user using an LED emitting visible light (e.g. 500 nm).

The mapping unit 21 may include additional sensors such as contact or proximity sensors, for example, the mapping unit 21 may comprise sensors for determining a skin characteristic, such as at least one of skin pigmentation, skin markings (i.e artificial or natural such as tattoo or moles), piercings, skin tone and moisture level.

The mapping unit 21 is functionally linked to memory 22, processor 23, user interface/feedback unit 24, and other circuitry (e.g. power supply 26) of apparatus 200. A bus 25 may connect the components of apparatus 200, as shown in Fig. 2.

Once the proposed treatment area is obtained by the mapping unit 21, the processor 23 divides the area into a plurality of treatment compartments or zones based on at least a surface area (dimensions) of the treatment applier 20. These compartments may or may not be of equal size. They may be discretely or continuously segmented. Other factors which could determine these compartments are the type of skin or body area (leg, arm, chest, etc.) currently proposed to be treated and/or the history of treatments on the area.

The pre-mapping of the treatment area allows the apparatus to accurately define treatment zones/compartments, which would all need to be treated for the processor 23 to determine that the treatment process has been completed successfully. Once completed, the processor 23 transmits a feedback signal to user interface unit 24 to prompt the user to move to a next location.

The processor 23 can calculate treatment parameters such as a number of treatments based on an area of the map and an area of the treatment applier 20. It can also calculate a speed of treatment based on the distance travelled by apparatus 200 in a period of time on the map.

The processor 23 may further determine an intensity of treatment and/or a location of a treatment compartment which is subjected to said intensity. A first set comprising each treated location X with the intensity used to treat location X may be stored in memory 22. As mentioned, apparatus 200 may comprise sensors which measure skin characteristics. Different skin characteristics require different treatment settings. A second set comprising each treated location X with the intensity used to treat location X and the determined skin characteristics may be stored in memory 22 to determine the right intensity settings for the skin characteristics. These correlations are not limiting, and memory 22 can stored more sets, or combine data from different sets. The monitoring of treatment parameters and the corresponding skin location helps not only ensure that uniform treatment settings are used by the user to treat the skin, but that correct intensity settings are used for the respective type of skin. In an aspect, based on the stored data sets, processor 23 can learn what kind of treatment settings would be applicable for a next compartment with a certain skin characteristic. Treatment parameters include, but are not limited to, in the context of this invention, a number of estimated treatments and/or time estimated to complete the number of estimated treatments, a number of applied treatments, speed of treatment applier 20, intensity of applied treatment, a position (compartment) of applied or to be applied treatment. They may be used to obtain a history of treatment on a skin or body area.

In an aspect, the processor 23 transmits a signal to the user interface unit 24 to prompt the user to position the treatment applier 20 of apparatus 200 substantially overlapping the reference position. The processor may register an arbitrary reference point in the obtained map, an arbitrary point of user contact or proximity in the boundary of the map or a start point of a manual trace as the reference position. The boundary may be highlighted to the user via the LED indicator for accurate alignment. The locations of treatment compartments with respect to the reference position are further determined.

The processor 23 determines that a treatment process has started when it registers contact or proximity of the treatment applier 20 at the reference position. It then guides the user through the treatment compartments or zones of the map by calculating displacement of the apparatus 200 relative to the reference position.

An orientation of the treatment applier 20 on the reference position and/or treatment compartments may further be determined by the mapping unit 21 (using e.g., the displacement sensor) by comparing a degree of an actual overlap of the treatment applier 20 with the reference position and/or the treatment compartment in the map.

In an aspect, the mapping unit 21 calculates displacement of the treatment applier 20 on the skin (and hence its position) from the reference position. The displacement is calculated by registering contact or proximity of the treatment applier on the map. The processor 23 uses this information to determine the extent of treatment, i.e., a number and location(s) of treated compartments. Based on the characteristics of the map stored in memory 22, processor 23 can determine a remaining number and location(s) of compartments to be treated in order to fill the map. The processor 23 further uses information stored by memory 22 on treatment parameters such as the intensity of treatment to determine whether a proposed compartment has already been subject to treatment. For example, processor 23 determines whether an intensity is stored in memory 22 for a particular treatment compartment where the treatment applier 20 is currently located. The processor 23 may further compare the actual number of treatments with an estimated number of treatments. The estimated number of treatments are obtained from the map characteristics such as area, speed of operation of apparatus 200 etc.

The memory 22 stores the map of the proposed treatment area and related information thereto, such as its boundary, the reference position, the number of estimated compartments, a previously treated compartment (position), a number of treatments required to fill the map, etc. In case of a light-based treatment apparatus, the number of treatments is the number of light flashes emitted towards the skin.

If the user deviates from the proposed treatment area, e.g. if processor 23 determines that contact or proximity between the treatment applier 20 and skin is lost at any moment of time during the estimated time to complete the number of treatments, it determines that a desired course of treatment has changed. Processor 23 transmits a feedback signal to the user interface unit 24 to provide feedback to the user about the change in status of treatment.

In an aspect, the user is prompted via the user interface unit 24 to position the treatment applier 20 on the reference position. A last registered displacement relative to the reference position is obtained by the processor 23 as the last treated compartment. In another aspect, processor 23 retrieves the reference position stored in memory 22, and directly prompts the user to the location of the next treatment compartment.

The processor 23 can be implemented in numerous ways, with software and/or hardware, to perform the various functions described herein. The processor 23 may comprise one or more microprocessors or digital signal processors (DSPs) that may be programmed using software or computer program code to perform the required functions and/or to control components of the processor 23 to effect the required functions. The processor 23 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The memory 22 can store data, information and/or signals (including image(s)) for use by the processor 23 in controlling the operation of apparatus 200 and/or in executing or performing the methods described herein. In some implementations the memory stores computer-readable code that can be executed by the processor 23 so that the processor 23 performs one or more functions, including the methods described herein. In particular embodiments, the program code can be in the form of an application for a smart phone, tablet, laptop, computer or server. The memory can comprise any type of non-transitory machine-readable medium, such as cache or system memory including volatile and nonvolatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The user interface unit 24 may comprise transceivers which enable a data connection to and/or data exchange with other devices, including any one or more of servers, databases, user devices, and sensors. It can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). It may further comprise circuitry to control any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

Apparatus 200 includes a power supply 26. If apparatus 200 is a self-contained device, power supply 26 may include a rechargeable or replaceable battery. Power supply 26 may further include a connection to an external power supply, such as to a power mains, transformer, converter, generator, charging unit, or other external power supply. Power supply 26 may provide electrical power for operation of mapping unit 21, treatment applier 20, memory 22, processor 23, user interface unit 24, or other component of apparatus 200.

It is known to the skilled person that apparatus 200 may include additional components such as elements necessary for the type of treated to be performed.

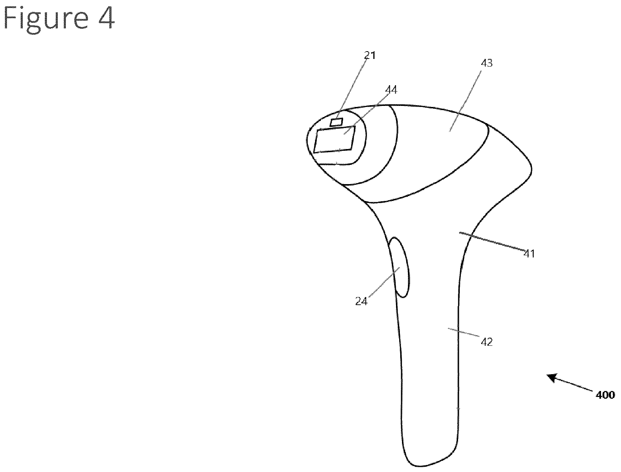

Fig. 4 shows an apparatus 400 which is a light-based (e.g. IPL) skin treatment apparatus, according to an embodiment of the invention.

Apparatus comprises a housing 41 that includes at least a handle portion 42 and a head portion 43. The handle portion 42 is shaped to enable the user to hold the apparatus 400 in a convenient manner. The head portion 43 includes treatment applier 44 that is to be placed into contact or proximity with skin in order for the treatment to be performed at the respective position.

The treatment may be carried out using light pulses (flashes). The treatment applier 44 comprises an optical window that is arranged in or on the housing 41 so that the window can be placed adjacent to or on the skin of the user.

Apparatus 400 further includes one or more light sources (not shown), e.g. inside the head portion 43, that are configured for generating light pulses to be applied to the skin of the subject via the window 45 and perform a treatment operation. The one or more light sources are arranged in the housing 41 so that the light pulses are provided from the one or more light sources through the window of treatment applier 44.

The one or more light sources can generate light pulses of any suitable or desired wavelength (or range of wavelengths) and/or intensities. For example, the light sources can generate visible light, infra-red (IR) light and/or ultraviolet (UV) light. Each light source can comprise any suitable type of light source, such as one or more light emitting diodes (LEDs), a halogen (Xenon) flash lamp, one or more lasers, etc. The light sources can provide light pulses with spectral content in the 530-1200 nm wavelength range.

Apparatus 400 further includes mapping unit 21, processor 23, user interface unit 24 and memory 22 which function in a similar manner as the general embodiment of Fig. 2.

The user interface unit 24 can be operated by the user to activate apparatus 400 so that the mapping and required treatment operation is performed on the body of the subject. Fig. 4 shows user interface unit 24 situated in the handle portion 42, but it could be situated at any suitable position on apparatus 400.

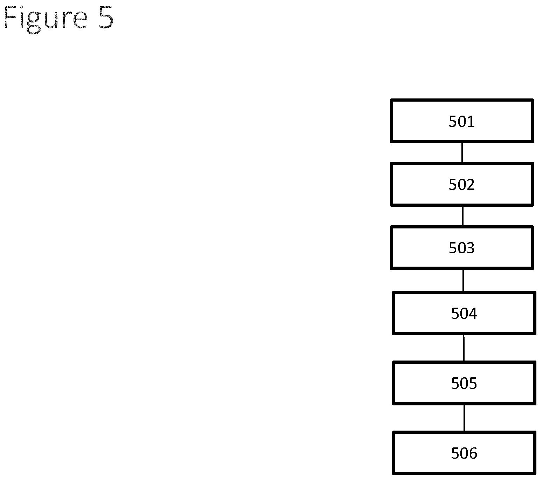

Fig. 5 shows a computer-implemented method of controlling apparatus 200 or 400 according to any of the above embodiments, to guide the user through the skin treatment process. In an aspect, the skin treatment process is light-based.

Step 501 comprises initializing the apparatus 200 or 400, so that the apparatus is ready for a skin treatment operation.

In step 502, the method includes obtaining a map of a skin surface to be treated. The map is obtained using mapping unit 21 in at least one manner as mentioned above. For example, the map is obtained by photographing/imaging the proposed treatment area using an imaging unit comprising a camera. Alternately or in addition, the map is obtained using action of a displacement sensor, based on a user trace. The method further includes determining a reference position on a boundary of the map.

In a preferred step 502, the map and its characteristics such as size, shape, reference position, are stored in a memory for use during the treatment process and/or thereafter as training data in case of a machine learning embodiment of processor 23.

In step 503, an area of the map is divided into treatment compartments based on at least an area or dimension of a treatment applier using which the treatment is performed by the user.

In a preferred step 504, the method includes calculating treatment parameters based on the map and/or the treatment applier. An intensity of treatment may further be recorded in the memory.

In step 505, the method comprises registering contact of the treatment applier at the reference position. This marks the start of treatment of skin which lies within the boundary of the map.

In step 506, the method guides the user through the compartments in the map based on displacement of the treatment applier relative to the reference position, till all compartments in the map are determined as having been treated.

All steps performed by the apparatus of 200 or 400 described above, are part of the method according to the invention.

According to yet another aspect of the invention, a computer program product is provided, which comprises a computer readable medium having computer readable code embodied therein, the computer readable code being configured such that, on execution by a suitable computer or processor, the processor is caused to perform the method described above.

It is possible to combine any feature mentioned as part of the above embodiments with a feature(s) of another embodiment, as long as the features are functionally and structurally compatible with each other. While the present disclosure has been described with the above-described exemplary embodiments, various changes and modifications may be suggested to one skilled in the art. It is intended that the present disclosure encompasses such changes and modifications as falling in the scope of claims.

## Claims

1. An apparatus (200, 400) for guiding a user during a skin treatment process comprising:
a treatment applier (20);
a mapping unit (21) configured to obtain a map of a skin area to be treated and a reference position on a boundary of the map, and
a processor (23) configured to:
divide an area of the obtained map into a plurality of compartments based on at least an area of the treatment applier,
register contact of the treatment applier at the reference position and
guide the user, via a user interface unit (24), through the compartments in the map based on displacement of the treatment applier (20) relative to the reference position.

2. The apparatus of claim 1, wherein the mapping unit (21) comprises a displacement sensor and/or an imaging unit.

3. The apparatus of claim 2, wherein the displacement sensor is an optical sensor or a mechanical sensor.

4. The apparatus of any of the preceding claims, further comprising a memory (22) for storing information relating to the map, the information comprising at least one of an area of the map, a number of compartments and the reference position.

5. The apparatus of any of the preceding claims, wherein the mappping unit (21) is configured to obtain the map via a manual trace and/or an imaging unit.

6. The apparatus of any of the preceding claims, further comprising a light source, and wherein the treatment applier (20) is adapted to apply a light-based treatment to the skin area.

7. The apparatus of claim 6, wherein the light source comprises a light emitting diode, laser, or a halogen flash lamp.

8. The apparatus of any of the preceding claims, wherein the processor (23) is further configured to calculate treatment parameters based on at least one of the map and the treatment applier (20).

9. The apparatus of claim 8, wherein the treatment parameters comprise at least one of a number of estimated treatments, a number of applied treatments, an intensity of an applied treatment, a position of a previously treated compartment, a position of at least one untreated compartment and a speed of the treatment applier.

10. The apparatus of any of the preceding claims,
wherein the processor is configured to determine, while guiding the user through the compartments, that a contact is lost between the treatment applier and the skin;
obtain a position of a last treated compartment and
indicate to the user, via the user interface unit (24), a position of a next compartment to be treated.

11. The apparatus of any of the preceding claims, wherein the user interface unit (24) comprises a visual indicator.

12. A computer-implemented method of guiding a user during skin treatment comprising the steps of:
obtaining a map of a skin surface to be treated and a reference position on a boundary of the map,
dividing an area of the obtained map into a plurality of compartments based on at least an area of a treatment applier,
registering contact of the treatment applier at the reference position and
guiding the user through the compartments in the map based on displacement of a treatment applier relative to the reference position.

13. The method of claim 12, further comprising calculating treatment parameters based on at least one of the map and the treatment applier.

14. The method of claims 12 or 13, wherein the skin treatment is based on treatment using light.

15. A computer program product, which comprises a computer readable medium having computer readable code embodied therein, the computer readable code being configured such that, on execution by a suitable computer or processor, the processor is caused to perform the method of any of claims 12-14.
